# EUROPEAN PATENT APPLICATION

(11) **EP 4 024 399 A1**
(43) Date of publication of application: **06.07.2022**
(21) Application number: 20217726.7
(22) Date of filing: 29.12.2020
(51) Int. Cl.: G16C 20/20, G06N 3/08, G06N 20/00

(54) **EARLY ODOR DETECTION**

(71) Applicant: Aryballe, 38000 Grenoble (FR)
(72) Inventor: Kantsingh, Krishna, 38950 Saint Marin Le Vinoux (FR); Caritu, Yanis, 38134 Saint-Joseph-de-rivière (FR); Rousselle, Tristan, 38320 Eybens (FR)
(74) Representative: Schuffenecker, Thierry

(57) **Abstract**

There is described a computer-implemented method comprising the steps of: receiving a stream of data from a multivariate sensor measuring responses of compounds or Volatile Organic Compounds (VOCs) in a sample; during absorption phase, not requiring equilibrium phase, determining the presence of one or more compounds based on one or more models trained by machine learning; wherein said machine learning is minimizing a loss function on features from said multivariate sensor determined during absorption, equilibrium and desorption phases of said one or more compounds. Developments comprise normalizing, using regression analysis such as (incremental) Linear Discriminant Analysis and/or metric learning, performing physical actions comprising purging the sensor, continuous monitoring and/or tracking predefined compounds over time, etc. System aspects are described, comprising the use of Neural Networks and/or of generic and/or specific sensors, and/or the use of a Mach-Zehnder Interferometer and/or of a Capacitive Micromachined Ultrasonic Transducer.

## Description

### Technical field

The document provides examples of signal processing, and in particular of machine learning methods and systems in the domain of odors' sensors.

### Background

An "odor" (American English) or "odour" (British English) is caused by Volatile Organic Compounds (VOCs) and/or molecules emitted by objects in the environment, which can be found in low concentrations in the air that humans and animals can perceive by their sense of smell. Other terms with different connotations can be used e.g. "smell", "scent", "aroma", "fragrance", "perfumes", etc.

Several VOCs (or compounds in a fluid) and/or molecules of interest can be present at the same time and mix altogether. This creates difficulties for gas recognition. This technical problem is often ignored in the scientific literature. Odors indeed can be in competition, from the user (or machine sensor) perspective. Volutes, turbulence or other fluid circulation also can lead to fluctuating values especially for magnitude or intensity characteristics of an odors.

Sensors in modern technology are becoming more and more complex. Systems of sensors contain a wide range of different sensors, where each may deliver a multitude of signals. Appropriate handling of data becomes crucial to reveal desired information.

The article "Real-time gas recognition and gas unmixing in a robot application" (by Maho and al. published in January 2020 reference HAL-02448737) discloses an optoelectronic nose which uses peptides as sensing materials, and Surface Plasmon Resonance (SPR) imaging as transduction method. There is described a real-time dictionary-based algorithm for dealing both with gas recognition and gas unmixing. Although real-time is discussed, this published approach presents limitations.

Known methods generally require to wait for an acquisition to finish, which is not ideal for *real time.* For example, the patent document WO2020141281 discloses a stability criterion to be met (over time) to obtain a characterization of the target. In other words, the stability parameter requires to be reached before the determination of an odor can be performed.

### Summary of the invention

There is described a computer-implemented method comprising the steps of: receiving a stream of data from a multivariate sensor measuring responses of compounds or Volatile Organic Compounds (VOCs) in a sample; during absorption phase, not requiring equilibrium phase, determining the presence of one or more compounds forming an odor based on one or more models trained by machine learning; wherein said machine learning is minimizing a loss function on features from said multivariate sensor determined during absorption, equilibrium and desorption phases of said one or more compounds. Developments comprise normalizing, using regression analysis such as (incremental) Linear Discriminant Analysis and/or metric learning, performing physical actions comprising purging the sensor, continuous monitoring and/or tracking one or more predefined compounds over time, etc. System aspects are described, comprising the use of Neural Networks and/or of generic and/or specific sensors, and/or the use of a Mach-Zehnder Interferometer and/or of a Capacitive Micromachined Ultrasonic Transducer.

Known approaches in the field of electronic nose require to wait for an acquisition to finish (which is not ideal for real time sensing). Integration, during the injection phase, also can require time delays, contrary to real-time criterion. Known methods generally do not extract valuable information from time-series, do not learn on interactions between compounds, and also do not take advantage of temporal evolutions of data, etc. Such analysis is not known or described in the state of the art.

Advantageously, embodiments of the invention allow not waiting for an acquisition to finish. This proves to be advantageous for "real time" sensing.

Advantageously, embodiments of the invention allow real-time detection which can be advantageous in specific contexts or environments, for example for the purpose of detecting explosives or drugs.

Advantageously, real-time sensing allows for continuous monitoring.

Advantageously, embodiments of the invention can cope with absorption phase only (or first desorption only, as some uses can start with desorption), not requiring equilibrium phase.

Advantageously, real-time or early detection allow purging (the electronic nose) earlier, which can preserve sensors from aging or from other deteriorations. An early purge indeed enables to safeguard sensor, because inefficient desorption (or excessive or inefficient absorption) can damage sensing units such as peptides (e.g. sticking molecules, contaminants, particulate matter e.g. a virus). Advantageously, embodiments of the invention can thus improve the lifetime of sensors.

Advantageously, early detection allows increasing the lifetime of the multivariate sensor. It reduces time exposure for a same given sensor, so that more measurement counts can be performed per given sensor. This generally can lead to minimize the downstream maintenance which can be expensive because of being time consuming (e.g. replacing a sensors' cartridge in a car given a use case of car sharing). Typically, instead of few tens of seconds or minutes required by known methods to detect one or more odors, system and methods according to the invention can reduce this time down to a few seconds.

Advantageously, real-time or early detection allow saving energy. Some embodiments indeed can comprise battery powering a laser source, valve and pump for purging, for example embedded in handheld devices (e.g. wearable computers, smartwatch) for which energy management is critical. Short acquisition and computation can save battery, and increase overall device' s autonomy. Embodiments related to Internet of Things (loT) also can present energy-critical situations (couples of seconds instead of minutes). Examples of steps can comprise early measurement and determination, switching off multivariate sensor e.g. purging or heating, thus preserving sensor lifetime along energy savings.

Advantageously, embodiments of the invention allow exploiting information from time-series, learning about interactions between compounds, taking advantage of temporal evolutions of data, etc.

Advantageously, embodiments of the invention allow using low-spec processors for real-time signal processing. Such processing units can be found in Internet of Things' devices for example. This advantage is enabled by efficient energy management.

### Brief description of drawings

Embodiments of the present invention will now be described by way of example with reference to the accompanying drawings in which like references denote similar elements, and in which:
FIG. 1 illustrates an example of a *sensorgram;*
FIG. 2 shows training univariate data, in the example outputs measured by one sensor unit over time;
FIG. 3 illustrates an embodiment of a machine learning mechanisms according to the invention;
FIG. 4 shows an example of an online mechanism once one or more models are learnt.

### Detailed description

An "electronic nose" (irrespective of sensor technology) produces a signal when its sensors interact with an odor. Based on the interaction of odor with sensors, the obtained signal is categorized into three different phases: a) absorption, b) equilibrium, c) desorption. Most often, after equilibrium phase, the obtained signal is transformed into a *signature* by performing the integration over time. In order to detect an odor, this pipeline requires to finish at-least equilibrium phase *and only then* a signature can be obtained for odor determination. These signal processing pipelines are not suitable for real-time monitoring of odors.

The structure of an electronic nose according to the invention can be diverse.

In an embodiment, the "electronic nose" or "analyzing system" comprises a measurement chamber intended to receive the target compounds contained in either gas, liquid or fluid sample, wherein a plurality of separate sensitive sites, each comprising receivers able to interact with the target compounds, is located in said chamber.

"Sensors" can be unidimensional (1D), bidimensional (2D) or tridimensional (3D). A sensor is a device that transforms a physical, chemical, or biological stimulus into a readable signal.

"Sample" designates gas (e.g. air) or liquids (e.g. human saliva).

"Target compounds" can be characterized by an electronic nose: compounds, VOCs, bacteria, virus, proteins, lipids, non-organic compounds, etc.

A "sensorgram" designates the temporal evolution of data signal, so as to extract interaction kinetics (adsorption and desorption) of target compounds with receptors.

A "sensogram" (without the letter "r") designates a graph of responses versus time in surface plasmon resonance studies. By extension, this term can be used hereinafter in other contexts.

### Real-time

Real-time is a consequence of the proposed methods and systems according to the invention.

In yet other words, said inference computation requires a duration which is small when compared to the acquisition process (typically comprising baseline, absorption, plateau (representative of equilibrium) and desorption.

The present methods and system according to the invention can deal with few first data points using pretrained models. Typically, inference (i.e. detection) can be performed as early as start of an absorption phase, or during absorption phase (or else desorption), but not requiring equilibrium phase. Contrary to known methods, there is no need to wait for the data acquisition to finish, no need for contextual data.

As a further consequence of fast inference (or real-time or early detection), the physical integrity sensor can be preserved, which increases the lifetime of the sensor, and also the energy consumption of the system (implying better battery autonomy), *infra.*

Real-time can refer to having *on-the-fly* inference/detection at each sampling point irrespective of their phase (small lag might appear owing to the computational and hardware limitations).

Real-time can imply an early detection. In other words, inference can be performed simultaneously to data acquisition. Inference can occur during measurement.

In other words, there is no need to wait for all responses. At each sampling, a response can be received and a detection/inference can be made.

"Real -time" detection designates the operation or step or task of performing with fast inference while maintaining a predefined minimal level of accuracy.

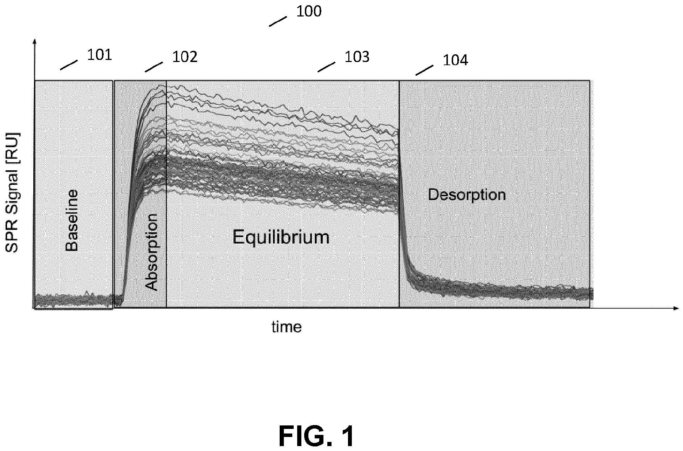

FIG. 1 illustrates an example of a *sensogram 100, in the example Surface Plasmon.* Different phases can be observed: adsorption 102 generally occurs exponentially, then a plateau 103 is obtained, then a desorption 104 occurs. In the absence of gas, the baseline 101 is measured.

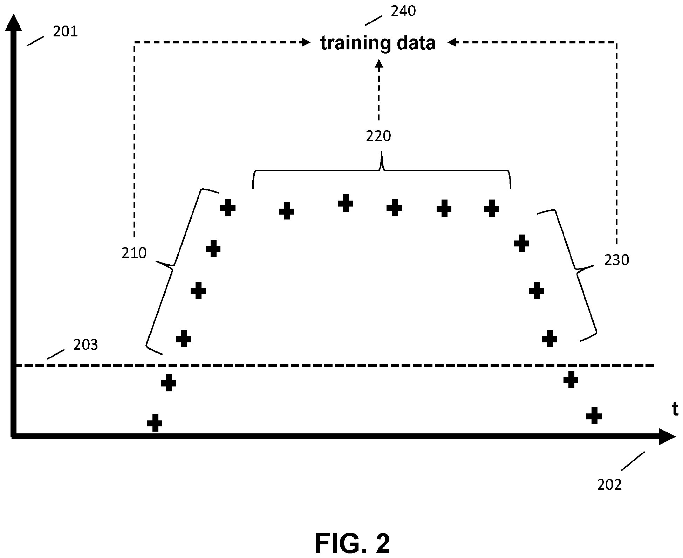

FIG. 2 shows training data, in the example outputs measured by one sensor 201 over time 202. In the example, a SPR imaging system is used. A threshold of measurement 203 exists. Data points (measures) are obtained over time. Different phases can be distinguished (absorption/adsorption 210, plateau or equilibrium 220 and desorption 230. These data points are used as training data 240, including all three phases.

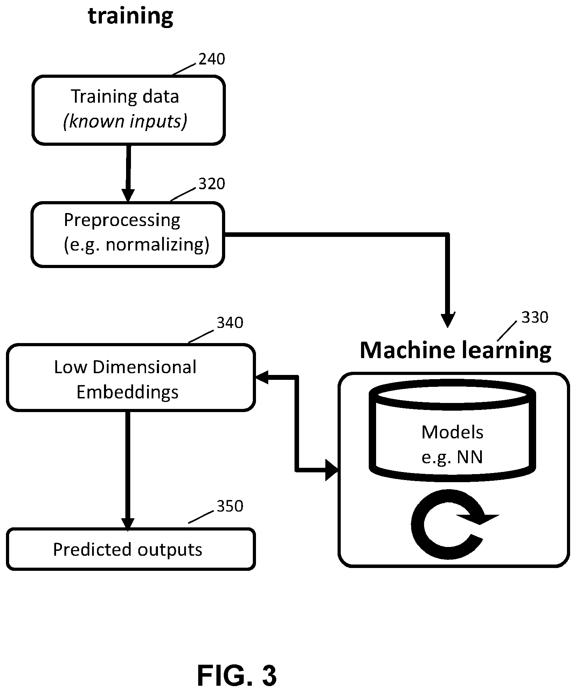

FIG. 3 illustrates an embodiment of machine learning mechanisms according to the invention. From known inputs 240, data is normalized 320 and low dimensional embeddings are extracted 340, by machine learning 330. In the context of neural networks, embeddings are low-dimensional, learned continuous vector representations of discrete variables. Neural network embeddings are useful because they can reduce the dimensionality of categorical variables and meaningfully represent categories in the transformed space. As a result, predicted outputs 350 associated with known inputs can be produced.

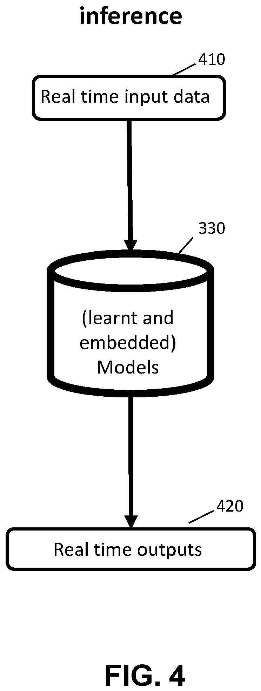

FIG. 4 shows the online mechanism once one or more models are learnt. Once machine learning 330 converges, low dimensional embeddings 350 can be embedded in objects, even small objects (e.g. Internet of Things), so as to provide outputs 420 from inputs 410. These predictions in particular can occur near real time. Also, few computing power is required. Embodiments of the invention thus comprise use cases such as: fire detection, bad odours in exhalations, sweat, cold cigarette smoke in rented cars, etc.

Various embodiments are now described.

In an electronic nose, VOCs (or fluid sample) are generally injected (passively and/or actively) into a chamber with or without inert gas (e.g. argon). Distinct sensor units (of distinct chemical affinity) forming a "multivariate sensor" in the chamber then react to compounds or VOCs or molecules of the fluid sample and from an analog output, a digital output is determined, and further interpreted.

Dimensions of an electronic nose according to the invention correspond to a few squared millimeters.

A "sensorgram" is obtained, comprising substantially parallel curves, each curve being the response of one sensor unit to compounds or VOCs. Amplifiers can be used.

From said sensorgram, a signature is determined (such as a radar chart), said signature being associated with one or more predefined "odors" (interpretation of the presence of compounds can be post-processed).

Databases can be built, in particular in the form of signatures and/or low-dimensional embeddings, i.e. using metric learning, PCA or trained neural networks.

Raw measurement outputs from the sensor may be the data effectively stored in the database even if they can benefit from some pre-processing before feeding a machine-learning system or method.

A sensor unit response generally comprises three phases. At first, only air or neutral inert gas is present: sensor units exhibit a "baseline" level. Then, VOCs start coupling with reagents and the "absorption" phase starts. At a certain moment, after a specific time duration, an "equilibrium" is obtained. Then, triggered by a purge (active removing of VOCs, caused by injecting air and/or other ways such as heating), the phase of "desorption" starts. Molecules leave sensor units (not necessarily symmetrical to the absorption phase). In some situations, the desorption phase can be the first and sometimes only step.

### Multivariate sensor (hardware embodiments)

Sensing can be performed using diverse materials or molecules. Below are listed various types of sensors which can be used in embodiments of the invention.

### Mach-Zehnder interferometer (MZI)

In an embodiment, the multivariate sensor comprises a Mach-Zehnder interferometer. Such an interferometer uses laser coherent pulses. One first beam or optical path traverses peptides and fluid sample, and is thus phase is changed. One second beam or optical path without change in phase then interferes with the first beam. Measures can then be interpreted.

In physics, a Mach-Zehnder interferometer is a device used to determine the relative phase shift variations between two guided coherent beams derived by splitting light from a single source. The interferometer can be used to measure phase shifts between the two beams caused by a sample or a change in length or refractive index of one of the paths.

In variants, the multivariate sensor comprises a Fizeau interferometer or a Fabry-Perot interferometer or a Jamin interferometer or a Ramsey-Borde interferometer.

In an embodiment, the sensor comprises an optical integrated circuit comprising: a lower layer called substrate; a first coupling means adapted to couple a radiation incident light to the integrated optical circuit; a directional splitter connected to the first coupling means and configured to separate the light radiation coupled by the first means from the second means coupling to at least one pair of waveguides comprising: a first waveguide known as a sensitive arm in which is propagated a first portion of the light radiation, said sensitive arm being exposed to a first ambient medium and at least one compound to be detected that induces a modification of the local refractive index perceived by the evanescent part of the electromagnetic field of the first portion of the light radiation, and a second waveguide called a reference arm in which is propagated a second portion of the light radiation, an encapsulation layer encapsulating the reference arm, said layer being impermeable to the compound(s) to be detected, so as to be able to detect that the reference arm is exposed only to a second medium the same nature as the first ambient environment, and devoid of said compound to be detected ; a directional combiner combining the first portion of the radiation from said reference arm, called the first transmitted portion, and the second portion of the light radiation from said sensitive arm, called the "sensitive arm" second transmitted portion, to form a transmitted radiation ; second coupling means adapted to couple said radiation; and transmitted to a medium external to the integrated optical circuit; an upper layer called superstrate covering at least the first and the second layers of the optical integrated circuit; second coupling means, the directional separator and the combiner directional and does not cover the sensitive and reference arm, said encapsulation layer being deposited on top of the superstrate.

In an embodiment, the sensitive arm and the reference arm are spiral waveguides. In an embodiment, the integrated optical circuit comprises a layer called functionalization layer. In an embodiment, the sensitive arm and the reference arm are spiral waveguides. In an embodiment, the integrated optical circuit comprises a layer called functionalization layer, at least partially covering the sensitive arm and adapted to adsorb one of the compounds to be detected ; In an embodiment, the optical integrated circuit comprises a so-called compensation layer covering at least partially the reference arm, a thickness of the compensation layer being equal to or higher than that of the compensation layer functionalization before adsorption of the compound to be detected. In an embodiment, the encapsulation layer is porous in the first medium of to allow a regulation of the hydrometric degree or of the osmotic pressure of the second medium relative to the first one ambient environment. In an embodiment, the encapsulating layer is made of glass, or silicon, polymer or metal. In an embodiment, the encapsulation layer is a deformable membrane. In an embodiment, an assembly formed by said directional separator, said reference arm, said encapsulation layer, sensing arm and combiner is called an interferometric set, the said optical circuit is called a directional optical circuit integrated with at least one additional directional separator configured to separate the light radiation coupled by the first means of coupling to a plurality of interferometric sets, and comprising a plurality of second coupling means, each comprising a plurality of second coupling means adapted to couple the radiation transmitted by an assembly different interferometric to a medium outside the optical integrated circuit. In an embodiment, the sensitive arm of each interferometric assembly includes a so-called functionalization layer covering at least partially the arm sensitive and adapted to adsorb one of the compounds to be detected, each functionalization layer being adapted to adsorb a compound to be used as a detect different from those adsorbed by the other layers of functionalization covering the sensitive arm of the others of sets Interferometric. In an embodiment, the reference arm of each interferometric assembly is encapsulated individually; the thickness of the individual encapsulation layer is less than 500 microns. In an embodiment, the reference arms of the interferometric assemblies are encapsulated collectively, so as to form a uniform encapsulation layer and without discontinuity. In an embodiment, the thickness of the individual encapsulation layer is between 5 microns and 2000 microns.

In an embodiment, the sensor comprises: a laser source configured to emit incident light radiation; an integrated optical circuit according to the invention; an optical detection system adapted to detect a light radiation; - an integrated optical circuit according to the invention from the second coupling means and generate a signal representative of the evolution over time of the detected light intensity; a unit for processing the said signal, adapted to determine, from the intensity detected, the evolution over time of the phase shift between the first portion and the second portion transmitted and the second portion transmitted.

### Capacitive Micromachined Ultrasonic Transducer, acronym CMUT

In an embodiment, the multivariate sensor according to the invention comprises a Capacitive Micromachined Ultrasonic Transducer, acronym CMUT.

CMUTs are the transducers where the energy transduction is due to change in capacitance. CMUTs are constructed on silicon using micromachining techniques. A cavity is formed in a silicon substrate, and a thin layer suspended on the top of the cavity serves as a membrane on which a metallized layer acts an electrode, together with the silicon substrate which serves as a bottom electrode.

If an AC signal is applied across the biased electrodes, the vibrating membrane will produce ultrasonic waves in the medium of interest (it works as a transmitter). If ultrasonic waves are applied on the membrane of a biased CMUT, it will generate alternating signal as the capacitance of the CMUT is varied. In this way, it works as a receiver of ultrasonic waves.

In an embodiment, the multivariate sensor can comprise a large number of CMUTs. In particular, CMUT-on-CMOS technology along flip-chip process can allow integration of CMUTs with front-end electronics.

### Surface Plasmon Resonance (SPR)

In some embodiments, the multivariate sensor comprises a microstructured chip for Surface Plasmon Resonance (SPR) analysis which is in the form of a solid consisting of a base, of an upper face, at least one part of which is covered with a metal layer, and of at least one side face, wherein said upper face is provided with zones of micrometric size intended to receive species to be analyzed chosen from n protuberances and m cavities; and in that when n+m≧2, said zones are separated from one another by planar surfaces, with n ranging from 1 to j and m ranging from 0 to i, j and i being integers. In a development, the n protuberances and m cavities have a curved surface with a radius of curvature R, preferably of between 0.1 and 600 mm. In a development, the n protuberances and m cavities have an identical radius of curvature. In a development at least one of the n protuberances and m cavities has a radius of curvature different from the others.

In some embodiments, the multivariate sensor comprises a microstructured chip for surface plasmon resonance (SPR) analysis which is in the form of a solid consisting of a base, of an upper face, at least one part of which is covered with a metal layer, and of at least one side face, in which said upper face is provided with zones having a size ranging from 1 µm to 1000 µm intended to receive species to be analyzed, chosen from n protuberances and m cavities; and when n + m z. 2, said zones are separated from one another by planar surfaces, said planar surfaces being parallel to a plane (XY), with n ranging from 1 to j, m ranging from O to i; j and i being integers; said zones having a curved surface with an average radius of curvature R located in a plane orthogonal to the plane (XY); and the average radius of curvature R of the curved surface is between 0.1 and 600 mm, characterized in that at least one of the n protuberances and m cavities has a radius of curvature different from the others.

### Other sensor embodiments

In an advantageous embodiment, peptides are used. Peptides are short chains of between two and fifty amino acids, linked by peptide bonds. Chains of fewer than ten or fifteen amino acids are called oligopeptides, and include dipeptides, tripeptides, and tetrapeptides. A "polypeptide" is a longer, continuous, unbranched peptide chain of up to approximately fifty amino acids. Hence, peptides fall under the broad chemical classes of "biological polymers and oligomers", alongside nucleic acids, oligosaccharides, polysaccharides, and others.

In some embodiments of the invention, polymers (on silicon or derivative materials like SiO2 or other traditional materials in micro-machining MEMS or silicon photonics) can be used. Silicon is a chemical element with the symbol Si and atomic number 14. It is a semiconductor.

In some embodiments, piezo resistance mechanisms can be used.

In some embodiments, living cells can be used.

In some embodiments, combinations of the above means can be used, e.g. a combination of peptides and polymers. Depending on use cases (i.e. general purpose or specific detection), the number and/or types of sensor units are diverse.

### Machine learning embodiments

### Generalities

Various types of machine learning are possible. Machine learning is a field of computer science that uses statistical techniques to give computer systems the ability to "learn" (e.g., progressively improve performance on a specific task) with data, without being explicitly programmed.

Machine learning is helpful for pattern detection and recognition. Advantageously, it's sometimes easier to collect the data than to explicitly write the program. In addition, neural networks can be revisited and retrained on new data; it doesn't need to be rewritten. Machine learning can advantageously be performed on "big data" *(infra),* i.e. using as much data as possible (stability, convergence, weak signals). New data is continuously added and training can be reiterated.

Machine learning tasks are typically classified into two broad categories, depending on whether there is a learning "label" or "feedback" or "annotation" available to a learning system:
"Supervised learning" designates a situation wherein the computer is presented with example inputs and their desired outputs, given by a "teacher", and the goal is to learn a general rule that maps inputs to outputs. As special cases, the input signal can be only partially available, or restricted to special feedback.

"Unsupervised learning" designates a situation wherein no labels are given to the learning algorithm, leaving it on its own to find structure in its input. Unsupervised learning can be a goal in itself (discovering hidden patterns in data) or a means towards an end (feature learning).

Other types of machine learning are:
"Semi-supervised learning" designates a situation wherein the computer is given only an incomplete training signal: training set with some (often many) of the target outputs missing.

"Active learning" designates a situation wherein the computer can only obtain training labels for a limited set of instances (based on a budget), and also has to optimize its choice of objects to acquire labels for. When used interactively, these can be presented to the user for labeling.

"Reinforcement learning" designates a situation wherein training data (for example in form of rewards and punishments) is given only as a feedback to the program's actions in a dynamic environment, such as driving a vehicle or playing a game against an opponent.

Deep learning (also known as deep structured learning) is part of a broader family of methods based on artificial neural networks with representation learning. It can be used either for unsupervised, semi-supervised or supervised learning (Wikipedia). Deep learning can be used advantageously in embodiments of the invention.

### Machine learning according to embodiments of the invention

Machine learning for odors can be very specific indeed.

Machine learning performed on images or videos can leverage large quantities of labeled meta data (annotation can be direct or indirect, e.g. by OCR, image recognition, contextual data) etc. By contrast, it is not possible to *store* odors (without transforming into a signal); moreover, annotation needs to be done immediately, as perception of an odor may change over time.

In addition, with respect to odor detection, the coupling between hardware and machine learning software is stronger: a measurement pipeline is specific, due to variations of the manufacturing. Two electronic noses can differ in the details. Following, machine learning is performed on data which is dependent, to a certain extent, on the considered hardware.

Machine learning according to the invention can be performed for a selection of sensing units (selection of peptides), with known inputs (by construction).

Frugal machine learning is possible. With respect to autonomous cars, the learning phase can use data collected from different types of hardware, yet the final embeddings can be portable. The same reasoning can be_applied for learning running by a user, with an accelerometer along other sensors. The same reasoning can occur for odor learning (contextualize acquisition, etc).

At stakes is the capability of grabbing and separating molecules that are specific and not interfering with one another. Schematically, it is the role devoted to the hardware to simplify the job for the software; the more specific is the hardware, the better is the situation for separating molecules with software algorithms.

By construction, in an embodiment, the training set is annotated. There is no limit to the size of the training dataset, only cost of investment. In other words, in such a situation, the confidence in annotations can be high.

### Machine learning according to the invention (LDA, QDA, Metric teaming)

In regression analysis, logistic regression is estimating the parameters of a logistic model (a form of binary regression). Mathematically, a binary logistic model has a dependent variable with two possible values, such as pass/fail which is represented by an indicator variable, where the two values are labeled "0" and "1".

In a development, Principal Component Analysis (PCA) can be used. PCA can be used for dimensionality reduction by projecting each data point onto only the first few principal components to obtain lower-dimensional data while preserving as much of the data's variation as possible.

In an embodiment, PCA can be used as a preprocessing step to Linear Discriminant Analysis (LDA).

In regression analysis, Linear Discriminant Analysis (LDA), normal discriminant analysis (NDA), or discriminant function analysis is a generalization of Fisher's linear discriminant, a method to find a linear combination of features that characterizes or separates two or more classes of objects or events. The resulting combination may be used as a linear classifier, or, more commonly, for dimensionality reduction before later classification.

In a development, incremental LDA can be used. The typical implementation of the LDA technique requires that all the samples are available in advance. However, there are situations where the entire data set is not available and the input data are observed as a stream. In this case, it is desirable for the LDA feature extraction to have the ability to update the computed LDA features by observing the new samples without running the algorithm on the whole data set. In real-time applications, the extracted LDA features can be updated as soon as new observations are available. Online local learning algorithms for updating LDA features incrementally can use error-correcting and Hebbian learning rules.

Quadratic discriminant analysis (QDA) is related to linear discriminant analysis (LDA), where it is assumed that the measurements from each class are normally distributed. Unlike LDA however, in QDA there is no assumption that the covariance of each of the classes is identical. Linear Discriminant Analysis refers to modeling and classifying the categorical response Y with a linear combination of predictor variables X. Quadratic Discriminant Analysis refers to modeling and classifying the categorical response Y with a non-linear combination of predictor variables X. LDA and QDA are often preferred over logistic regression when we have more than two non-ordinal response classes. LDA is a less flexible classifier than QDA. QDA can be recommended if the training set is large.

Regarding regression analysis, models which can be used in embodiments of the invention can comprise one or more of : linear regression, simple regression, polynomial regression, general linear model, generalized linear model, discrete choice, binomial regression, binary regression, logistic regression, multinomial logit, mixed logit, probit multinomial probit, ordered logit, ordered probit, Poisson multilevel model, fixed effects, random effects, linear mixed-effects model, nonlinear mixed-effects model, nonlinear regression, nonparametric, semiparametric, robust quantile, isotonic, principal components, least angle, local segmented, Errors-in-variables

### Metric learning

Advantageously, metric learning algorithm can be used for classification and embeddings. Results obtained by metric learning are as good as embodiments implementing LDA (although different embeddings). Advantageously, LDA is significantly faster to train and presents low computational complexity.

"Metric Learning" or "distance metric learning" designates the task of learning a distance function over objects. A metric or distance function has to obey four axioms: non-negativity, identity of indiscernibles, symmetry and subadditivity (or the triangle inequality). In practice, metric learning algorithms ignore the condition of identity of indiscernibles and learn a pseudo-metric. Based on data, the metric learning problem is generally formulated as an optimization problem where one seeks to find the parameters of a distance function that optimize some objective function measuring the agreement with the training data.

The learned distance metric can then be used to perform various tasks (e.g., k-NN classification, clustering, information retrieval).

Metric learning algorithms comprise linear distance and similarity learning. It is also possible to learn nonlinear metrics or multiple linear metrics, multi-task and semi-supervised learning. Metric learning can be applied for structured data like strings, trees, graphs and time series. Algorithms generally learn so-called *Mahalanobis* distances. Metric learning can include learning from relative comparisons based on Triplet loss, Neighborhood component analysis, Large margin nearest neighbor, Information theoretic metric learning (ITML).

Distance metric learning is related to similarity learning.

### Online machine learning

In an embodiment, machine learning is carried out offline (e.g., in laboratory or manufacturing stage). Yet in an embodiment, machine learning is carried out online. Machine learning indeed can be performed incrementally, or online. When the model is known (stabilized weights in neural networks) and embedded, it is possible to continue learning a data flow (to improve the existing model, without starting from scratch). When an offline machine learning learns on a complete data set, an online learning can continue learning ("learning transfer"), in an embedded way, without having to re-insert the initial data. Advantageously, the learning can be done in advance (basic, pre-requisite) and then customized on company-specific or pilot-specific data.

### Federated learning

Federated learning is a machine learning technique which trains an algorithm across multiple decentralized edge processing units or servers holding local data samples, without exchanging them. By contrast, in centralized approaches local datasets are uploaded to one server, or local data samples are identically distributed. Federated learning enables multiple entities to build a common, robust machine learning model without sharing data. One particular advantage of this approach is that it can respect data privacy and/or data security, and/or data access rights.

The general principle consists in training local models on local data samples and exchanging parameters (e.g. the weights and biases of a deep neural network) between these local nodes at some frequency to generate a global model shared by all nodes.

Distributed learning originally aims at parallelizing computing power where federated learning originally aims at training on heterogeneous datasets. While distributed learning also aims at training a single model on multiple servers, a common underlying assumption is that the local datasets are identically distributed and roughly have the same size. None of these hypotheses are made for federated learning; instead, the datasets are typically heterogeneous and their sizes may span several orders of magnitude. Moreover, the clients involved in federated learning may be unreliable as they are subject to more failures or drop out since they commonly rely on less powerful communication media (i.e. Wi-fi) and battery-powered systems (i.e. smartphones and loT devices) compared to distributed learning where nodes are typically datacenters that have powerful computational capabilities and are connected one another with fast networks.

### Deep learning

In an embodiment, machine learning according to the invention (software and hardware) comprises deep learning, wherein pre-training is unsupervised. Depending on embodiments, the human contribution in machine learning steps can vary. In some embodiments machine learning is applied to machine learning ("automated machine learning"). The entire process can be automated indeed, including using multiple models and comparing results. In most cases, humans are involved in machine learning ("Human in the loop"). Data engineers or developers are responsible for maintaining the data pipeline: ingesting data, cleaning data, feature engineering, and model discovery (roles of a teacher, a curator of training data, and an analyst of results). In "deep learning" for example, data scientists seek for the right neural network architectures and parameters.

### Big data

In one embodiment, large collections of data can be collected (expression "Big data"). Big data is associated with a number of technical concepts, comprising volume (large collections, even if redundant), variety (stemming from numerous sources), velocity (constant updating of data in dynamic environments), veracity (i.e., how much noise is in the data, embedding of weak signals that may not be seen otherwise) and value (insights have technical or business uses).

In one embodiment, large collection of data, including metadata (data about data) can be collected across a plurality of electronic noses or odor sensors according to embodiments of the invention.

### Connectivity

Processing can be performed locally and/or remotely (distance resources). Connectivity can be continuous or intermittent. In some embodiments, network access can be available and processing (inference) can be performed remotely (e.g. elastic cloud along local processor in the smartphone). In some embodiments, it may well be that no connection is accessible (e.g. loT device): databases can be used locally (low embeddings). Local databases or neural networks also enables fast responses time.

Various embodiments are now described.

There is described a computer-implemented method comprising the steps of: receiving a stream of data from a multivariate sensor measuring responses (or data signals) of Volatile Organic Compounds in a gas, a liquid or an aqueous phase; during absorption phase, not requiring equilibrium phase, determining/inferring the presence of one or more compounds or VOCs forming an odor based on one or more models trained by machine learning; wherein said machine learning is minimizing a loss function on features from said multivariate sensor determined during (a plurality of complete) absorption phases, equilibrium phases and desorption phases of one or more compounds VOCs.

Data signals or responses are received from multivariate sensor. Compounds binding to the receptors of the multivariate sensor restitute or cause or provoke or provide response(s). The more concentrated, the higher the intensity's response(s). Data is multivariate in a multivariate sensor.

There is described a computer-implemented method comprising the steps of: receiving a stream of data from a multivariate sensor measuring responses of Volatile Organic Compounds (in a gas) or compounds (in a fluid), binding to said multivariate sensor, in a gas, a liquid or an aqueous phase; at each time step (of the computation), determining a confidence level in the inference for determining a presence of one or more compounds or VOCs based on one or more models trained by machine learning; upon the confidence level in the inference exceeding a predefined threshold and/or ranges of thresholds, determining the presence of one or more VOCs forming an odor; wherein said machine learning is minimizing a loss function on features from said multivariate sensor determined during a plurality of absorption phases, equilibrium phases and desorption phases of one or more compounds or Volatile Organic Compounds. In a development, the predefined threshold is set so that the step of determining the presence of one or more compounds or VOCs occurs during absorption phase (only). In a development, the threshold is dynamically adjusted.

In a development, the step of determining the presence of one or more compounds is an inference step. In a development, said inference step does not need to wait for the data acquisition to finish. In a development, said inference is performed at each sampling point irrespective of its phase. In a development, said inference step can be performed simultaneously to data acquisition or measurement.

The preceding technical effects ("real-time", "early", "simultaneous", etc) are surprising consequences of the specific machine learning being operated on the specific sensor hardware, as described. Pretrained models, i.e. trained according to embodiments of the invention, exhibit such properties, which can in turn be leveraged for useful usages (e.g. monitoring, tracking, saving energy, increasing lifetime of sensors).

Four fundamental states of matter comprise solid, gas, liquid and plasma. Fluid is a phase of matter and includes liquids, gases and plasmas.

Absorption is generally the first phase, followed by equilibrium and then desorption. In rare cases, the cycle can start by desorption phase (for example by eating). At this point, properties of the machine learning according to hardware embodiments of the invention allow determination of present compounds or VOCs.

An odor is a combination of VOCs.

"Features" designate the result of recombination and filtering of raw outputs stemming from sensing units.

It is implicit that machine learning is performed over several (or "a plurality of") if not many (complete time series responses or sensor grams or responses). It is not required to explicit precisely how many are required. The more data, the better. This is inherent to machine learning. The fact that the specified type of machine learning works particularly well, because / due to hardware specifics is not known.

Multi-sensor data from multivariate sensor enables multivariate analysis. Multivariate analysis MVA is based on the principles of multivariate statistics, which involves observation and analysis of more than one statistical outcome variable at a time. Typically, MVA is used to address the situations where multiple measurements are made on each sensor unit and the relations among these measurements and their structures are important.

In a development, the method further comprises a step of preprocessing raw data stemming from the multivariate sensor, for example normalizing said data and generating new features.

The step of normalizing after/during machine learning allows learning separating compounds instead of learning concentrations. There is generally no information at equilibrium: signature does not change. Normalizing allows removing or cancelling the effect of concentration or dilution.

In an embodiment, data is not normalized before machine learning is applied. This causes the models to learn also about concentrations, in some situations.

Data stemming from the sensor(s) also can be classified or categorized ("smells good" or "smells bad"), in which case the learnt models apply on such classes.

In an embodiment, the particular combination of a "fluidic-less" sensor and/or a MZI sensor handled by LDA shows good experimental results.

In an embodiment, metric learning shows good experimental results for monitoring or tracking. Low dimension embeddings can follow the dynamics on the evolution of compounds or VOCs. Metric learning increases the discrimination power of a machine learning algorithm.

In a development, the step of determining the presence of one or more compounds or VOCs comprises using regression analysis.

In an embodiment, regression analysis can comprise Linear Discriminant Analysis and/or Quadratic Discriminant Analysis. LDA (or QDA) experimentally show better generalization than other algorithms such as decision tree or random forest.

In a development, machine learning is online machine learning, e.g. using incremental Linear Discriminant Analysis.

Online machine learning is a method of machine learning in which data becomes available in a sequential order and is used to update the best predictor or classifiers for future data, by contrast with "batch" or "offline" machine learning (learning on the entire training data set at once). Advantageously, algorithms can dynamically adapt to new patterns in the data. ILDA can be a sequential ILDA or a chunk ILDA. An incremental LDA method also can comprise label propagation.

In a development machine learning comprises metric learning.

"Metric Learning" learns a distance function over objects. The learned distance metric can then be used to perform various tasks (e.g., k-NN classification, clustering, information retrieval). Metric learning can be performed before LDA.

In an embodiment, machine learning comprises federated learning. Federated learning advantageously provides a robust machine learning model without sharing data, or with limited sharing (data privacy and/or data security).

In an embodiment, machine learning uses one or more techniques of the list comprising Random Forest, Support Vector Machines, Adaptive Boosting, Back-Propagation Artificial Neural Network and/or Convolutional Neural Network.

In an embodiment, the method further comprises a step of semi-supervised anomaly detection, wherein the step of semi-supervised anomaly detection comprises using one or more of the methods or techniques comprising statistical techniques, density-based techniques such as k-nearest neighbor, local outlier factor or isolation forests, subspace and correlation-based outlier detection for high-dimensional data, Support Vector Machines, Bayesian Networks, Hidden Markov models, Cluster analysis-based outlier detection, Fuzzy logic-based outlier detection, Minimum Covariance Determinant or a combination thereof.

In one embodiment, the method further comprises a step of semi-supervised anomaly detection.

In data mining, anomaly detection (also outlier detection) designates the identification of rare items, events or observations which raise suspicions by differing significantly from the majority of the data (e.g. bank fraud, structural defects, problems or errors in a text). Anomalies are also referred to as *outliers, novelties, noise, deviations and exceptions.*

In an embodiment, a "semi-supervised" anomaly detection technique is used by determining a model representing normal behavior from a given normal training data set, and then testing the likelihood of a test instance to be generated by the learnt model. The term "semi" means that one or more human users can be involved at some step of the process (human experts or "noses").

In one embodiment, the step of semi-supervised anomaly detection comprises using one or more methods or techniques comprising statistical techniques, density-based techniques such as k-nearest neighbor, local outlier factor or isolation forests, subspace and correlation-based outlier detection for high-dimensional data, Support Vector Machines, replicator neural networks, Bayesian Networks, Hidden Markov models, Cluster analysis-based outlier detection, or Fuzzy logic-based outlier detection.

The minimum covariance determinant (MCD) estimator is a robust estimator of multivariate location and scatter. It can be computed efficiently; hence, a suitable algorithm for anomalies detection. In an embodiment, the method further comprises the step of displaying one or more determined comparisons or anomalies, and triggering one or more visual and/or vibratile and/or audio alerts depending on the application of predefined thresholds on said one or more comparisons or anomalies. If the difference is within safe boundaries, no alert is triggered. If outside safe boundaries (inferior to low bound, superior to upper bound), then an alert can be triggered. If the situation is borderline (e.g. plus or minus a given tolerance to the envelope frontiers), then an invitation to perform verifications can be triggered. Both inputs and outputs can be validated.

In a development, the step of determining is performed in real-time, that is during a time duration that occurs during absorption, or else desorption, but not requiring equilibrium phase.

In an embodiment, the method enables an "early detection" or "real-time" detection of compounds or VOCs. The detection can deal with desorption only, or absorption only. The expression "Real -time" detection designates the operation or step or task of performing detection in real time (short delays of time compared to acquisition), with fast inference. "Real-time" designates a time duration that occurs at the start of absorption phase (or first desorption), thus eliminating the need of equilibrium phase (even for compounds or VOCs where equilibrium is hard to obtain).

In a development, the method further comprises performing one or more physical actions upon determining the presence of one or more predefined compounds or VOCs and/or determining an anomaly, said physical actions comprising injecting air, switching on/off a valve, or purging the multivariate sensor.

In a development, the method further comprises, switching off a valve if associated to the multivariate sensor and/or purging the multivariate sensor after one or more compounds or VOCs are determined.

Such physical actions allow preserving the battery and/or the multivariate sensor. This implies that the autonomy of the embedded battery, if any, can be increased. The lifetime of the multivariate sensor can be increased.

In some embodiments, the sample of compounds or VOCs being measured can be expensive or otherwise hazardous; it is therefore advantageous to perform measurements quickly and with few limited amounts of matter.

In an embodiment, the method comprises the steps of: receiving a stream of data from a multivariate sensor measuring responses of compounds in a gas, a liquid or an aqueous phase; during absorption phase, determining the presence of one or more compounds based on one or more models trained by machine learning; upon (or as soon as or when or immediately after or substantially after) determining the presence of one or more predefined compounds, performing one or more physical actions, said physical actions comprising one or more of the actions comprising: injecting air, switching on/off a valve, purging the multivariate sensor, actuating an actuator, opening a bottle, sending a message, or initializing an action or procedure ; wherein said machine learning is minimizing a loss function on features from said multivariate sensor determined during a plurality of absorption phases, equilibrium phases and desorption phases of one or more compounds.

In an embodiment, the method comprises the steps of: receiving a stream of data from a multivariate sensor measuring responses of compounds in a gas, a liquid or an aqueous phase; determining the presence of one or more compounds based on one or more models trained by machine learning; upon (or as soon as or when or immediately after or substantially after) determining the presence of one or more predefined compounds, performing one or more physical actions.

In an embodiment, the method comprises the steps of: receiving a stream of data from a multivariate sensor measuring responses of compounds in a gas, a liquid or an aqueous phase; during absorption, or before equilibrium (which can be qualified as such when signals are stabilizing), determining the presence of one or more compounds based on one or more models trained by machine learning; upon determining the presence of one or more predefined compounds, performing one or more physical actions.

In an embodiment, the method comprises the steps of: receiving a stream of data from a multivariate sensor measuring responses of compounds in a gas, a liquid or an aqueous phase; during absorption phase (which does not require to be finished), continuously or regularly at predefined time intervals determining one or more compounds (e.g. the presence or the concentrations thereof) based on one or more models trained by machine learning; determining (e.g. values or presence of) one or more compounds. In a development, the method further comprises the step of performing one or more physical actions (e.g. immediately, i.e. as soon as (the presence and/or concentrations) of one or more compounds is known or determined), e.g. with sufficient confidence.

If the P (VOC | signal) is superior to a threshold, measurement can be stopped, wherein P is predicted probability for a VOC (or VOCs) given the signal, by a machine learning model. Machine learning models can output probabilities (or confidence) as their predictions. In most cases, VOCs are easy to predict and the predefined threshold may be crossed at the start of absorption phase, hence measurement can be stopped early. On the contrary, some VOCs might require more data to reach or excess the predefined threshold. Whenever the machine learning models are unable to reach the predefined threshold, this can indicate either incremental training or human intervention (anomaly detection).

In a development, the method further comprises a step of continuously monitoring the gas, liquid or aqueous phase, by reiterating steps of receiving a stream of data, determining the presence of one or more compounds or VOCs by machine learning, and/or a step of tracking one or more predefined compounds or VOCs over time.

It can be advantageous indeed to monitor one or more compounds or VOCs over time (dynamic aspect of evolution over time and/or space). For example, it can be advantageous to follow for how long an odor will last, or evolve (e.g. perfume, wine).

In an embodiment, the method comprises the step of following trajectories of data points and comparing those to known odors or reference levels or trajectories. The dynamics over time can be monitored (for example to measure the efficiency of deodorants).

In a development, the method further comprises a step of displaying data associated with the determined one or more compounds or VOCs.

In a development, one or more of the intermediate computation results and/or the computation context of one or more steps of the method is displayed in a man-machine interface.

Human-machine interaction is indeed complex. A feedback loop may be "closed" i.e. inaccessible to human control (it is executed by the machine). It can be "open" (e.g. display step in a human-machine interface, validation or any other human confirmation system). Different embodiments can lead to different implementations by closing, respectively opening, one or more feedback loops. In this way, the process (which may be "artificial intelligence") can be interpreted as "transparent" in the sense of controllable. The display can relate to intermediate computation results, information about root causes, and/or the computation context. In this way, embodiments can be considered "explainable artificial intelligence".

The system implementing steps of the method can comprise one or more displays in a man-machine interface.

In an embodiment, "to display an odor" can mean to re-actuate said odor by delivering some compounds associated with an odor (odor "replay").

In an embodiment, the verb "to display" means to display on a screen (e.g. one or more of a symbol, an icon, a color, a numerical value, etc)

Man-machine interfaces can include one or more selection interfaces (by pointer, via a mouse and/or trackpad and/or joystick, by voice command, by gloves or glove, etc), graphic tactile interfaces, voice interfaces, gesture and position interfaces.

Various display devices can be used. The display can be placed on a smartwatch, or in a smartphone. Display means also can comprise an opaque virtual reality headset or a semitransparent augmented reality headset or a headset with configurable transparency, projectors (pico-projectors for example, or video-projectors to project simulation scenes) or a combination of such devices. The display can be placed in or on a "head-mounted display", a "wearable computer", "glasses", a headset, etc. The information displayed can be entirely virtual (displayed in the individual helmet), entirely real (e.g. projected on the flat surfaces available in the real cockpit environment) or a combination of both (partly a virtual display superimposed or merged with reality and partly a real display via projectors).

There is described a system for early odor detection comprising: a trained Neural Network, implemented in software and/or in hardware, configured to receive a stream of data from a multivariate sensor sensing compounds or Volatile Organic Compounds in a gas or a fluid; during absorption phase, not requiring equilibrium phase, inferring, by a processor, the presence of one or more Volatile Organic Compounds forming an odor, said trained neural network encoding one or more models determined by machine learning; wherein the training of said neural network is performed by regression analysis on data signals or responses stemming from said multivariate sensor during a plurality of (e.g. cycles, repeated measurements) absorption, equilibrium and/or desorption phases of one or more Volatile Organic Compounds.

A trained neural network can be seen as a "database". Such databases are legally protected (*sui generis*), as investments. In perfumes, users may want to store signatures of compounds of theirs. Databases can be used by man and/or machine.

In a development, the neural network comprises one or more of: a network of artificial neurons; an acyclic artificial neural network; a recurrent neural network; a forward-propagating neural network; a convolutional neural network; a network of generative antagonistic neurons.

Some types of neural networks allow/require learning to be "supervised" by the operator, while others operate independently. Some types operate purely in hardware, while others are purely software and run-on general-purpose computers.

There is disclosed a system comprising deep neural networks and/or deep belief networks and/or recurrent neural networks adapted to carry out one or more of the steps of the method. Software Neural network simulators can be used (software applications simulating the behavior of artificial or biological neural networks). Artificial or biological neural networks can be used (physical NN, e.g. ADALINE, Physical Neural Network, Phase change neural network, Memristive neural network).

In an embodiment, recurrent neural network(s) can be used. A recurrent neural network (RNN) is a class of artificial neural networks where connections between nodes form a directed graph along a temporal sequence. This allows it to exhibit temporal dynamic behavior.

In an embodiment, Long Short-Term Memory (LSTM) is used. LSTM is an artificial recurrent neural network (RNN) architecture used in the field of deep learning. Unlike standard feedforward neural networks, LSTM has feedback connections. It can efficiently process both partial and entire sequences of streaming data.

In an embodiment, Gated Recurrent Units (GRUs) are used. GRUs are long short-term memory (LSTM) with a forget gate, but with fewer parameters than LSTM, as it lacks an output gate.

In an embodiment, Hidden Markov Model (HMM) can be used. A Hidden Markov Model (HMM) is a statistical Markov model in which the system being modeled is assumed to be a Markov process with unobservable ("hidden") states. If the HMM is used for time series prediction, sophisticated Bayesian inference methods, like Markov chain Monte Carlo (MCMC) sampling can be used.

Neural Network can be implemented in software and/or hardware. For example, TensorFlow can convert a Neural Network into deployable software C and/or Javascript code, that can for example be implemented on a microcontroller (or on a web browser/ on a cloud).

In a development, the multivariate sensor comprises one or more generic sensors and/or one or more specific sensors.

Generic sensors can be sensitive to a plurality of known (and unknown) molecules. Specific sensors react to predefined known molecules or compounds (for example NH or OH), allowing to identify ammonia or water, providing background noise.

In an embodiment, the multivariate sensor comprises N sensing units sensitive to M distinct types of compounds or VOCs, with N>M and O sensing units sensitive to P distinct types of compounds or VOCs, with O<P.

Such an arrangement comprises both specific and generic sensors, which can avoid background noise, while presenting high discriminability.

In a development, the multivariate sensor comprises a Mach-Zehnder Interferometer and/or a Capacitive Micromachined Ultrasonic Transducer.

In a development, the multivariate sensor is fluidic-less, e.g. with no pump and no valve.

In an embodiment, the injection is active (one or more pumps with one or more valves). Micro or nano pumps (respectively valves) can be used. Using a pump allows to accumulate molecules. In situations where there is no pump, there is a need for confined environment and/or time for measurement (e.g. approaching the sensor towards the source of odors). In an embodiment, the injection is performed with a pump (e.g. micro-pump) and no valve. In an embodiment, the sampling is fluidic-less: only diffusion, no pump, no valve; for example, by Pelletier effect, fluid convections enable measurements (e.g. accumulation and then desorption by heating).

In an embodiment, the system comprises one or more pre-concentrators (µPC).

A concentrator us a fully optional accessory to be used with electronic nose for increasing the limit of detection, maintaining consistent headspace for sampling and/or removing effect of background signal (i.e. signal of water or ethanol etc.).

In an embodiment, the multivariate sensor comprises a couple of sensing units which are specific to certain common compounds such as ethanol, water, or ammonia. The presence of a high background signal, such as water vapor from aqueous samples, can deteriorate discriminability. For example, odors coming from a yoghurt comprise water and other smells. It is advantageous to remove the smell associated with humidity, in order to distinguish other compounds. As another example, odors coming from a perfume comprise ethanol and fragrances. It can be advantageous to remove the smell associated with ethanol.

In an embodiment, the multivariate sensor is associated with (e.g. coupled to) a pre-concentrator (µPC). A pre-concentrator system comprises a glass or metal tube packed with powder adsorbent and a large oven (often larger than the sensor itself), which can require energy to heat up quickly.

In addition to a gain of selectivity provided by the use of the µPC, other mechanisms of VOC concentration can also play a role in the differentiation ability for the flavored samples since it can allow us to lower the detection limit of the targeted compounds or VOCs.

In an embodiment, a miniaturized pre-concentrator can be packed with an optional hydrophobic adsorbent (to improve the detection limit of gas analysis methods, and to enhance selectivity by reducing the background signal).

In an embodiment, the system comprising the multivariate sensor coupled with the pre-concentrator can integrate a hygrometer, for humidity measurements of the sensed gas. In order to deal with water interference removing, one or more hydrophobic sensors, such as modified polyaniline, can be used.

In a development, the multivariate sensor system is integrated in a consumer electronics device, or for example associated with the cork of a bottle.

Consumer electronics devices comprise but are not limited to: smartphone, smartwatch, wearable computer, head-mounted computer, smart glasses, kitchen appliance such as fridge or microwave oven, coffee machine, laptop or generally a computer.

In an embodiment, the multivariate sensor is integrated in the cork of a bottle. Energy can be supplied via a battery (internal or external) and/or by induction (query on demand). Advantageously, evolution over time of a bottle of wine or liquor can thus be monitored. The assembly can be disposable or reusable, at least in part.

In an embodiment, various physical actions can be triggered upon particular measurements and determinations performed based on said measurements. For example, a flashing LED can be triggered if a problem is detected in a (e.g. wine) bottle. A fluid can be injected in the bottle.

A valve can be opened or closed. In other words, a retroaction or feedback can be triggered based on the detection of one or more compounds or VOCs.

The disclosed methods can take form of an entirely hardware embodiment (e.g. FPGA), an entirely software embodiment or an embodiment containing both hardware and software elements. Software embodiments include but are not limited to firmware, resident software, microcode, etc. The invention also can take the form of a computer program product accessible from a computer-usable or computer-readable medium providing program code for use by or in connection with a computer or any instruction execution system. A computer-usable or computer-readable can be any apparatus that can contain, store, communicate, propagate, or transport the program for use by or in connection with the instruction execution system, apparatus, or device. The medium can be an electronic, magnetic, optical, electromagnetic, and/or semiconductor system (or apparatus or device) or a propagation medium or the like.

There is disclosed a computer program comprising instructions for carrying out one or more of the steps of the method when said computer program is executed on a computer device.

## Claims

1. A computer-implemented method comprising the steps of:
- receiving a stream of data from a multivariate sensor measuring responses of compounds in a gas, a liquid or an aqueous phase;
- during absorption phase, not requiring equilibrium phase, determining the presence of one or more compounds based on one or more models trained by machine learning;
wherein said machine learning is minimizing a loss function on features from said multivariate sensor determined during a plurality of absorption phases, equilibrium phases and desorption phases of one or more compounds.

2. The method of Claim 1, further comprising a step of preprocessing raw data stemming from the multivariate sensor, for example normalizing said data.

3. The method of Claim 1, wherein the step of determining the presence of one or more compounds comprises using regression analysis.

4. The method of any preceding claim, wherein machine learning is online machine learning, e.g. using incremental Linear Discriminant Analysis or Bayesian learning.

5. The method of any preceding claim, wherein machine learning comprises metric learning.

6. The method of any preceding claim, further comprising performing one or more physical actions upon determining the presence of one or more predefined compounds and/or determining an anomaly, said physical actions comprising injecting air, switching on/off a valve, or purging the multivariate sensor.

7. The method of any preceding claim, further comprising a step of continuously monitoring the gas, liquid or aqueous phase, by reiterating steps of receiving a stream of data, determining the presence of one or more compounds by machine learning, and/or further comprising a step of tracking one or more predefined compounds or VOCs over time.

8. The method of any preceding claim, further comprising the step of displaying data associated with the determined one or more compounds.

9. The method of any preceding claim, wherein the steps of determining the presence of one or more compounds is an inference step, and wherein said inference step does not need to wait for the data acquisition to finish, or is performed at each sampling point irrespective of its phase, or wherein said inference step can be performed simultaneously to data acquisition or measurement.

10. A system for early odor detection comprising:
- a trained Neural Network, implemented in software and/or in hardware, configured to receive a stream of data from a multivariate sensor sensing compounds in a gas, a liquid or an aqueous phase;
- during absorption phase, not requiring equilibrium phase, inferring, by a processor, the presence of one or more compounds, said trained Neural Network encoding one or more models determined by machine learning;
wherein the training of said neural network is performed by regression analysis on data signals stemming from said multivariate sensor during a plurality of absorption phases, equilibrium phases and desorption phases of one or more compounds.

11. The system of Claim 10, wherein the trained Neural Network is encoding a trained history-dependent model, associated with one or more of Long Short-Term Memory, Gated Recurrent Units and/or Hidden Markov Model receiving time series data from said multivariate sensor.

12. The system of Claim 10, wherein the multivariate sensor comprises one or more generic sensors and one or more specific sensors.

13. The system of Claim 10, wherein the multivariate sensor comprises a Mach-Zehnder Interferometer and/or a Capacitive Micromachined Ultrasonic Transducer.

14. The system of Claim 10, wherein the multivariate sensor is fluidic-less, e.g. with no pump and no valve.

15. The system of Claim 10, wherein the multivariate sensor system is integrated in a consumer electronics device, or for example associated with the cork of a bottle.
